# NEW EUROPEAN PATENT SPECIFICATION

(11) **EP 1 581 184 B2**
(45) Date of publication and mention of the opposition decision: **09.09.2020**
(45) Mention of the grant of the patent: 24.08.2011
(21) Application number: 03775738.2
(22) Date of filing: 08.12.2003
(51) Int. Cl.: A61K 9/06, A61K 9/10, A61P 31/04

(54) **VETERINARY COMPOSITIONS FOR TREATING MASTITIS**
ZUSAMMENSETZUNGEN ZUR TIERÄRZTLICHEN BEHANDLUNG VON MASTITIS
COMPOSITIONS VÉTÉRINAIRES DESTINÉES AU TRAITEMENT DE LA MAMMITE

(30) Priority: 20.12.2002 GB 0229642
(43) Date of publication of application: 05.10.2005
(73) Proprietor: Zoetis UK Limited, London EC4A 3AE (GB); Zoetis LLC, Florham Park NJ 07932 (US)
(72) Inventor: DORGAN, Roderick John Jeremiah, Pfizer Global R&D, Kent, CT13 9NJ (GB)
(74) Representative: Mannion, Sally Kim
(86) International application number: PCT/IB2003/005825
(87) International publication number: WO 2005/051352

(56) References cited:
- EP-A- 0 271 306
- EP-A2- 0 001 090
- WO-A1-94/13261
- WO-A1-98/26759
- WO-A1-03/022245
- GB-A- 1 441 747
- GB-A- 1 456 346
- GB-A- 1 456 349
- GB-A- 1 547 164
- GB-A- 2 273 441
- GB-A- 2 273 441
- GB-A- 2 273 443
- US-A- 3 252 859
- US-A- 3 912 806
- US-A- 4 011 312
- US-B1- 6 254 881
- US-B1- 6 254 881

## Description

The present invention relates to a veterinary composition for the prophylaxis and treatment of mastitis in mammals. More particularly, the present invention relates to the treatment of mastitis in cows.

It is well established that bacterial infection via the teats of a cow is the most common cause of mastitis. Various treatments are available in the prior art which attempt to prevent mastitis occurring or to treat the symptoms of mastitis. Thus, teat dip compositions are disclosed inUS 5211961, which are used as antibacterial washes for cleaning the teats. Alternatively, EP 1104233 and EP 799047 disclose film-forming compositions which can be applied to the teat to provide a barrier to the entry of bacterial agents.

Another approach employed involves the provision of a physical barrier in the teat canal in order to prevent the ingress of pathogens as described in GB 1441747. GB 2273441 discloses an antibacterial formulation and a seal formulation which are used in combination to form a plug and treat mastitis. A related patent, GB 2273443, likewise discloses the use of an antibacterial formulation and a seal formulation for the treatment of mastitis in cows. In GB 2273441, the seal formulation comprises a gel base and a non-toxic heavy metal salt in the base whereas in GB 2273443 the seal formulation comprises a polyethylene gel. The antibiotic is provided separately in an aqueous formulation and GB 2273441 discloses that the aqueous formulations of antibiotic lead to rapid absorption in a very short time period.

Surprisingly, we have now found that the co-administration of a vegetable oil-based product with a seal formulation containing a non-toxic heavy metal salt leads to an improvement in seal effectiveness. There are various types of oil such as mineral oil, silicone oil and vegetable oil and we have found that the use of a vegetable oil based antibiotic in combination with a seal formulation leads to an improvement in the seal effectiveness.

The tests conducted in GB 2273441 indicated that an aqueous-based antibiotic formulation provided significant advantages relative to prior art formulations in the treatment of dry cows. We have now found that, in fact, the use of oil-based formulations, and in particular vegetable oil-based formulations of antibiotic, leads to further improvements insofar as consumer health and animal welfare are concerned.

An important property of an ideal teat seal is that the seal should be capable of remaining in situ for the duration of the dry cow period. The seal should also have sufficient integrity such that it is not caused to break or rupture within the udder. In addition, the seal formulation should be compatible with the co-administered formulation. The formulations of the present invention satisfy these criteria and lead to improved performance of the seal and improved teat condition.

The exact reason for the improved performance and teat condition of animals treated with formulations according to the present invention is not fully understood. Furthermore, the prior art unambiguously indicates that aqueous based antibiotic formulations are rapidly absorbed and provide significant advantages. Without wishing to be bound by theory, it is believed that the vegetable oil-based formulations of the present invention enhance the effectiveness of the seal formulation in situ and offer improved compatibility with the seal in the teat canal relative to aqueous formulations. It is believed that the physical properties such as the density, and viscosities of the vegetable oils also are in some way connected with the improvement in performance.

The formulations of the present invention comprise two parts which are generally administered to the animal as two separate formulations. The first part is a seal formulation comprising a gel base and a non-toxic heavy metal salt in the base. The second part of the formulation is a peanut oil or peanut oil and hydrogenated peanut oil-based product which may for instance comprise an antibiotic in a peanut oil or peanut oil and hydrogenated peanut oil-based formulation. The oil-based formulation may however contain any pharmaceutically active agent for the treatment or prophylaxis of disease in cattle.

According to one aspect of the present invention, there is provided a veterinary composition for intra-mammary use in non-human animals comprising an antibacterial formulation and a separate seal formulation, wherein the antibacterial formulation is a peanut oil or peanut oil and hydrogenated peanut oil-based formulation and the seal formulation comprises a gel base and a non-toxic heavy metal salt in the base in an amount of at least 30% by weight of the gel base.

In an embodiment of the present invention, the heavy metal is preferably bismuth, especially as the sub-nitrate salt. Preferably, the heavy metal salt is present in an amount of from 40% to 80% by weight of the gel base and more preferably in the range of from 50% to 70% by weight. A particularly effective seal formulation contains about 65% by weight ie from 62 to 66% by weight of the heavy metal salt.

In another embodiment of the invention, the base is a gel based on aluminium stearate and more preferably the gel also includes a vehicle such as liquid paraffin.

In another embodiment of the invention, the co administered peanut oil or peanut oil and hydrogenated peanut oil based formulation contains an antibiotic. We have found peanut oil and hydrogenated peanut oil to be particularly effective as the base for an antibacterial formulation which is to be used in conjunction with a seal formulation.

In another embodiment of the present invention, the antibacterial agent is preferably a beta- lactam antibiotic such as a penicillin or cephalosporin. More especially, cloxacillin and more preferably cloxacillin benzathine. However, any antibiotic which has been approved for veterinary use may be employed.

In another embodiment of the present invention, the veterinary composition of the present invention is provided as a unit dose. Typically, a unit dose would contain 600 mg of cloxacillin in the form of cloxacillin benzathine.

In an alternative aspect of the present invention, the antibacterial formulation and the seal formulation may be provided as a single formulation which may be administered to the teat canal of a non-human animal directly in one step. The relative proportions of the ingredients in such a formulation are identical to those in a formulation which is to be applied as two separate formulations.

In a further aspect of the present invention, there is provided the use of a veterinary composition comprising an antibacterial formulation and a seal formulation either separately or in a single formulation as defined above in the manufacture of a medicament for the treatment of prophylaxis or mammary disorders in non-human animals.

Examples of specific seal formulations which may be used with the oil-based antibiotic formulations of the present invention are disclosed in GB 2273441 and GB 2273443

For the avoidance of doubt, suitable seal formulations and specific examples of seal formulations disclosed in those documents are suitable for use in the formulations of the present invention and are included within the scope of seal formulations according to the present invention.

The integrity of veterinary formulations according to the present invention was assessed by administration into the quarters of a dry cow. The cow was dosed with three different vegetable oil-based intra-mammary antibiotic formulations, each placed in a separate teat. Each of the three teats was then sealed with a bismuth-based teat seal containing 65% by weight bismuth sub-nitrate as described in formulation 2A4 of GB 2273441. The fourth teat received only the seal formulation having an identical seal formulation (ie containing 65% by weight bismuth sub-nitrate) but no antibiotic formulation. The teats were assessed by X-ray analysis and the radioopacity of the heavy metal salt provided clear pictures of the seal integrity and amount of seal within the desired area of the teat canal. The teats were X-rayed at days 1, 7, 14 and 21 to determine the area of opacity and hence the seal integrity. The results are summarised in Table 1 below.

**Table 1**

| | TEAT 1 | TEAT 2 | TEAT 3 | TEAT 4 |
|---|---|---|---|---|
| ANTIBIOTIC FORMULATION | None | Formulation A | Formulation B | Formulation C |
| AVERAGED AREA OF OPACITY | 1.0 | 1.1 | 1.7 | 1.6 |

Antibiotic formulation A contained peanut oil and hydrogenated peanut oil. Antibiotic formulations B and C contained peanut oil. In each case, it was observed that co-administration of the vegetable-oil-based antibiotic formulation in conjunction with the seal formulation lead to an increase in the amount of seal present at the base of the teat canal. Thus, despite an expectation that the oil-based antibiotic formulation might disperse the oil-based seal formulation it was in fact observed that the amount of seal present in the base of the teat increased. Thus, the formulations of the present invention offer advantages relative to prior art formulations. It is important that the seal is present at the base of the teat and that it remains intact at the base of the teat in order to prevent the ingress of organisms into the udder which will lead to mastitis.

The veterinary formulation of the present invention satisfied these requirements.

## Claims

1. A veterinary composition for intra-mammary use in non-human animals comprising an antibacterial formulation and a separate seal formulation, wherein the antibacterial formulation is a peanut oil or peanut oil and hydrogenated peanut oil-based formulation and the seal formulation comprises a gel base and a non-toxic heavy metal salt in the base in an amount of at least 30% by weight of the gel base.

2. A composition as claimed in claim 1, wherein the heavy metal is bismuth.

3. A composition as claimed in claim 2, wherein the heavy metal salt is bismuth sub-nitrate.

4. A composition as claimed in claim 1, 2, or 3, wherein the heavy metal salt is present in an amount of from 40% to 80% by weight of the gel base.

5. A composition as claimed in any preceding claim, wherein the base is a gel based on aluminium stearate.

6. A composition as claimed in any preceding claim, wherein the vegetable oil base for an antibacterial formulation is peanut oil and/or hydrogenated peanut oil.

7. A composition as claimed in any preceding claim, wherein the antibacterial agent is a beta-lactam antibiotic.

8. A composition as claimed in claim 7, wherein the antibacterial agent is cloxacillin.

9. Use of a veterinary composition comprising an antibacterial formulation and a seal formulation separately as defined above in any of claims 1 to 5 in the manufacture of a medicament for the treatment of prophylaxis or mammary disorders in non-human animals.

## Patentansprüche

1. Veterinäre Zusammensetzung zur intra-mammären Verwendung bei nicht-menschlichen Tieren, umfassend eine antibakterielle Formulierung und eine getrennte Versiegelungsformulierung, wobei die antibakterielle Formulierung eine Erdnussöl- oder Erdnussöl und hydriertes Erdnussöl-basierte Formulierung ist und die Versiegelungsformulierung eine Gelgrundlage und ein nicht-toxisches Schwermetallsalz in der Grundlage in einer Menge von wenigstens 30 Gew.-% der Gelgrundlage umfasst.

2. Zusammensetzung, wie sie in Anspruch 1 beansprucht ist, wobei das Schwermetall Bismut ist.

3. Zusammensetzung, wie sie in Anspruch 2 beansprucht wird, wobei das Schwermetallsalz Bismutsubnitrat ist.

4. Zusammensetzung, wie sie in Anspruch 1, 2, oder 3 beansprucht ist, wobei das Schwermetallsalz in einer Menge von 40 bis 80 Gew.-% der Gelgrundlage vorliegt.

5. Zusammensetzung, wie sie in einem vorangehenden Anspruch beansprucht ist, wobei die Grundlage ein Gel ist, das auf Aluminiumstearat basiert.

6. Zusammensetzung, wie sie in einem vorangehenden Anspruch beansprucht ist, wobei die Pflanzenölgrundlage für eine antibakterialle Formulierung Erdnussöl und/oder hydriertes Erdnussöl ist.

7. Zusammensetzung, wie sie in einem vorangehenden Anspruch beansprucht ist, wobei das antibakterielle Mittel ein beta-Lactam-Antibiotikum ist.

8. Zusammensetzung, wie sie in Anspruch 7 beansprucht ist, wobei das antibakterielle Mittel Cloxacillin ist.

9. Verwendung einer veterinären Zusammensetzung, umfassend eine antibakterielle Formulierung und eine Versiegelungsformulierung, entweder getrennt oder in einer einzelnen Formulierung, wie oben in einem der Ansprüche 1 bis 5 definiert, bei der Herstellung eines Medikaments zur Behandlung oder Prophylaxe von mammären Störungen bei nicht-menschlichen Tieren.

## Revendications

1. Composition vétérinaire pour utilisation intramammaire chez des animaux non humains, comprenant une formulation antibactérienne et une formulation d'étanchéité séparée, dans laquelle la formulation antibactérienne est une formulation à base d'huile arachide ou d'huile arachide et d'huile arachide hydrogénée et la formulation d'étanchéité comprend un gel de matière de base et un sel de métal lourd non toxique dans la matière de base en une quantité d'au moins 30 % en poids du gel de base.

2. Composition suivant la revendication 1, dans laquelle le métal lourd est le bismuth.

3. Composition suivant la revendication 2, dans laquelle le sel de métal lourd est le sous-nitrate de bismuth.

4. Composition suivant la revendication 1, 2, ou 3 dans laquelle le sel de métal lourd est présent en une quantité de 40 % à 80 % en poids du gel de matière de base.

5. Composition suivant l'une quelconque des revendications précédentes, dans laquelle la matière de base est un gel à base de stéarate d'aluminium.

6. Composition suivant l'une quelconque des revendications précédentes, dans laquelle l'huile végétale de base pour une formulation antibactérienne est l'huile d'arachide et/ou l'huile d'arachide hydrogénée.

7. Composition suivant l'une quelconque des revendications précédentes, dans laquelle l'agent antibactérien est un antibiotique du type bêta-lactame.

8. Composition suivant la revendication 7, dans laquelle l'agent antibactérien est la cloxacilline.

9. Utilisation d'une composition vétérinaire comprenant une formulation antibactérienne et une formulation d'étanchéité soit séparément soit dans une formulation unique répondant à la définition précitée dans l'une quelconque des revendications 1 à 5 dans la production d'un médicament destiné au traitement ou à la prophylaxie de troubles mammaires chez des animaux non humains
